(19) Europäisches Patentamt
European Patent Office
Office européen des brevets

(11) Publication number: 0 033 215 A2

# (12) EUROPEAN PATENT APPLICATION

(21) Application number: 81300251.6

(22) Date of filing: 21.01.81

(51) Int. Cl.³: C 07 D 249/22
A 61 K 31/41

(30) Priority: 23.01.80 GB 8002327

(43) Date of publication of application: 05.08.81 Bulletin 81/31

(84) Designated Contracting States: BE CH DE FR GB LI NL

(71) Applicant: BEECHAM GROUP LIMITED
Beecham House Great West Road
Brentford, Middlesex(GB)

(72) Inventor: Buckle, Derek Richard
18 Hillfield
Redhill Surrey(GB)

(72) Inventor: Tedder, John Martin
40 Horley Road
Redhill Surrey(GB)

(74) Representative: Dawson, Hugh Bainforde et al,
Beecham Pharmaceuticals Great Burgh Yew Tree
Bottom Road
Epsom Surrey KT18 5XQ(GB)

## (54) Process for preparing anti-allergy compounds.

(57) Process for the preparation of compounds of the formula (I):

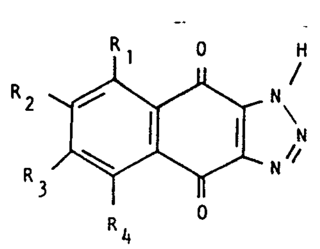

(I)

and pharmaceutically acceptable salts thereof wherein $R_1$, $R_2$, $R_3$ and $R_4$ which may be the same or different, represent hydrogen, halogen, nitro, lower alkyl or lower alkoxy, where lower means of up to six carbon atoms, or any adjacent two of $R_1$ to $R_4$ taken together represent an alkylene group containing from 3 to 5 carbon atoms or a 1,4-buta-1,3-dienylene group, by removing an N-protecting group Q from a compound of formula (II):

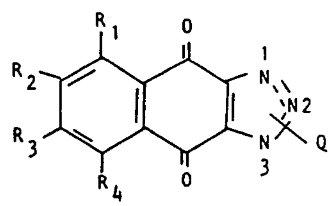

(II)

EP 0 033 215 A2

PROCESS FOR PREPARING ANTI-ALLERGY COMPOUNDS

This invention relates to a process for preparing anti-allergy compounds.

European Patent Application No. 78300485.6 published 13th June, 1979 and allowed US Patent Application Serial No. 953,464 disclose that compounds of the formula (I);

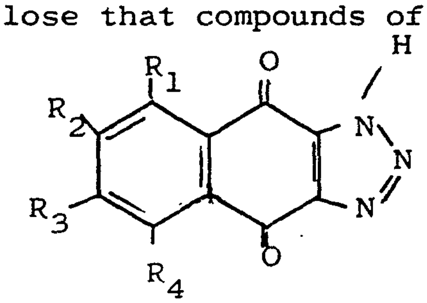

(I)

and pharmaceutically acceptable salts thereof wherein $R_1$, $R_2$, $R_3$ and $R_4$ which may be the same or different represent hydrogen, halogen, nitro, lower alkyl or lower alkoxy, where lower means of up to six carbon atoms or any adjacent two of $R_1$ to $R_4$ taken together represent an alkylene group containing from 3 to 5 carbon atoms or a 1,4-buta-1,3-dienylene group, are of value in the prophylaxis of diseases in which the symptoms are controlled by mediators of the allergic response. Examples of such diseases include bronchial

asthma, rhinitis, hayfever and allergic eczema.

These European and US Patent Applications also describe a process for the preparation of the compounds of the formula (I).

A new process for the preparation of the compounds of the formula (I) has now been discovered.

Accordingly, the present invention provides a process for the preparation of a compound of the formula (I) as hereinbefore defined, or a pharmaceutically acceptable salt thereof, which process comprises deprotecting a compound of formula (II):

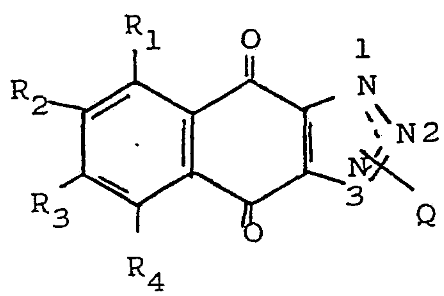

(II)

wherein Q is a N-protecting group at N-1 or N-3 and the other variables are as defined.

Suitable examples of Q groups are labile benzyl groups such as $C_{1-6}$ alkoxy substituted benzyl groups, for example 4-methoxybenzyl, 2,4-dimethoxybenzyl or 2,4,6-trimethoxybenzyl.

Such Q protecting groups may be removed from a compound of the formula (II) in any convenient manner, for example by acid catalysis. It is preferable to use the 4-methoxybenzyl protecting group which is readily removed using trifluoroacetic acid, the course of the cleavage being followed by NMR spectroscopy. Suitably temperatures of around 20-80$^{\circ}$C can be used, with a suitable reaction

time being around 3-4 hours. Other strong acids such as methanesulphonic acid behave similarly.

Compounds of the formula (II), which as novel intermediates form an important part of this invention, may be prepared by reacting a compound of the formula (III):

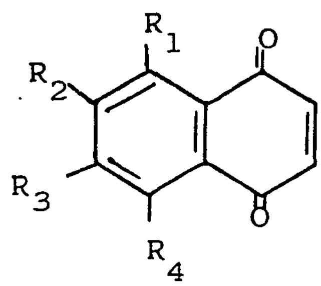

(III)

with an azide $QN_3$.

It will be appreciated from the foregoing that a particularly preferred azide will be 4-methoxybenzyl azide.

This reaction may be carried out by reaction of the compound of formula (III) with the azide at temperatures between 30 and 80°C, preferably 50°C, in a suitable organic solvent such as N,N-dimethylformamide (DMF), ethyl acetate or toluene. Preferably DMF is the solvent, more preferably ethyl acetate. The reaction time is dependent on the solvent and temperature but is usually in the order of 3 - 24 hours.

With unsymmetrical quinones a mixture of N-1 and N-3 benzyl naphthotriazoles will usually be formed. These may be sepa may be separated by conventional techniques such as column chromatography if required or deprotection as a mixture to give a single N-H naphthotriazole as a common product.

It will be appreciated that although it is believed that the process of this invention is applicable to the preparation of any compound of the formula (I), it will be especially useful in the preparation of compounds of the formula (I) that we stated in the European and US

Patent Applications to be of particular interest.

Such compounds are as follows:

One group is that in which at least one of $R_1$ to $R_4$ is hydrogen and the remainder are as previously defined. An example of such a compound is 4,9-dihydro-6,7-dimethyl-5-nitro-4,9-dioxo-1H-naphtho[2,3-d]-triazole. A further group is one where two of $R_1$ to $R_4$ are hydrogen and the remainder are as previously defined. An example of such a compound is 4,9-dihydro-5,6-dimethyl-4,9-dioxo-1H-naphtho-[2,3-d]-triazole. One preferred sub-group of compounds of formula (I) is that in which $R_1$ and $R_4$ are hydrogen and $R_2$ and $R_3$, which may be the same or different, represent methyl, ethyl or n-propyl.

In the European and US Patent Applications the compound 4,9-dihydro-6,7-dimethyl-4,9-dioxo-1H - naphtho-[2,3-d]-v-triazole, and its salts - especially its sodium salt - are highlighted. In a preferred embodiment of this invention the process is adapted for the preparation of this compound, or of its salts. This adaptation is readily achieved by use of a compound of formula (II) wherein $R_1$ and $R_4$ are hydrogen, and $R_2$ and $R_3$ are both methyl.

As disclosed in the European and US Patent Applications, the triazole moiety of the compounds of formula (I) has an acidic hydrogen, and accordingly may form salts, including pharmaceutically acceptable salts. These include aluminium, alkali metal and alkaline earth metal salts such as the sodium, potassium and magnesium salt; and salts with organic bases such as amines or amino compounds including physiologically active amines such as (-) ephedrine. The sodium and (-) ephedrine salts are preferred.

Salts may be made by conventionally reacting the compound of the formula (I) with the relevant metal or oxide or a base or basic salt thereof as appropriate or with the relevant base as appropriate. Salts may be inter-converted by passage through a suitable ion-exchange column.

The following Examples illustrate the process of this invention:

Example 1

(a) 4,9-Dihydro-6,7-dimethyl-4,9-dioxo-1(6-methoxybenzyl) naphtho[2,3-3]-v-triazole (D.1)

6,7-Dimethylnaphtho-1,4-quinone (2.0 g, 0.0108 mole) and p-methoxybenzyl azide (0.49 g, 0.030 mole) were dissolved in DMF (25 ml) and the mixture stirred at 50°C for 18 hours. The mixture was cooled and the DMF removed using a rotary film evaporator. The brown residue (2.7 g) was chromatographed on silica gel (Merck 7729) eluting with 3:7 chloroform:light petroleum (60°-80°) to give 0.157 g (15.3%) of the title compound Mpt: 178-9°; Analysis: Calculated : C, 69.16; H, 4.93; N, 12.1; Found C, 68.45; H, 4,83; N, 11.99: Nmr: δ ($CDCl_3$): 8.1 (s, 1H); 8.0 (s, 1H); 7.55 (d, 2H); 6.9 (d, 2H); 6.0 (s, 2H); 2.4 (s, 6H).

The same reaction effected in ethyl acetate resulted in a 42% yield of the title compound.

(b) Deprotection of (D.1) with trifluoroacetic acid to give 4,9-dihydro-6,7-dimethyl-4,9-dioxo-1H-naphtho [2,3-d]-v-triazole.

4,9-Dihydro-6,7-dimethyl-4,9-dioxo-1(p-methoxybenzyl-naphtho[2,3-d]-v-triazole (155 mg, 0.45 mole) was dissolved in trifluoroacetic acid (TFA)(5 ml). After 8 hours at 25° the solution was diluted with an equal volume of water when a solid precipitated, this was filtered off and discarded. The aqueous TFA liquors were treated with more water (30 ml) which yielded another precipitate. This was filtered off and dried to give the title compound as its monohydrate (64 mg, 60.8%), Mpt: 239-45° (physical data identical to authentic material; cf EP 78300485.6 or USSN 953,464).

Example 2

(a) 4,9-Dihydro-4,9-dioxo-1(4-methoxybenzyl)naphtho[2,3-d]-v-triazole (D.2)

1,4-Naphthoquinone (1.20g, 0·0075 mole) and 4-methoxybenzylazide (0.40g,0.0025 mole) were dissolved in ethyl acetate (50ml) and the mixture refluxed for five hours and then cooled overnight. The solution was shaken with a solution of ferric chloride (0.5g in 50ml of 0.2N HCl). The ethyl acetate was separated, washed with water (1x50ml), dried and evaporated. The residue was purified by chromatography on silica gel, gradient eluting with ethyl acetate/light petrol b.p. 60-80°. Naphthoquinone was recovered first (0.710g, 59.2%) followed by the title compound (0.431 g, 55.1%); Mpt 194°; NMR ($CDCl_3$) δ 3.8 (s, 3H, $OCH_3$); 5.96 (s, 2H, $PhC\underline{H}_2$); 7.2 (AB, dd, J = 9Hz, Δν = 54Hz, 4H); 7.8 (m, 2H); 8.3 (m, 2H, $C_5$-$C_8$H's).

(b) Deprotection of 4,9-dihydro-4,9-dioxo-1(4-methoxybenzyl)naphtho-[2,3-d]-v-triazole with trifluoroacetic acid to give 4,9-dihydro-4,9-dioxo-1H-naphtho-[2,3-d]-v-triazole.

4,9-Dihydro-4,9-dioxo-1(4-methoxybenzyl)naphtho-[2,3-d]-v-triazole (0.025g) was dissolved in trifluoroacetic acid (2ml) and the solution heated to 50°. After 5 hours the mixture was cooled and the solvent evaporated. The residue was treated with hydrochloric acid (5N,5ml) and the mixture again evaporated to dryness. This residue was taken

up in a mixture of ethyl acetate (5 ml) and sodium hydroxide solution (N, 5 ml). After shaking the phases were separated and the ethyl acetate layer was extracted with water (3 ml). The combined aqueous phases were acidified to pH 1 with hydrochloric acid and extracted with ethyl acetate (2 x 11 ml). The organic layers were combined, dried and evaporated to yield 1,4-dihydro-1,4-dioxonaphtho-[2,3-d]-v-triazole (0.014 g, 89.7%); Mpt 250°, NMR identical to authentic material (Mpt 250°); cf EP 78300485.6 or USSN 953,464.

Claims

1. A process for the preparation of a compound of the formula (I):

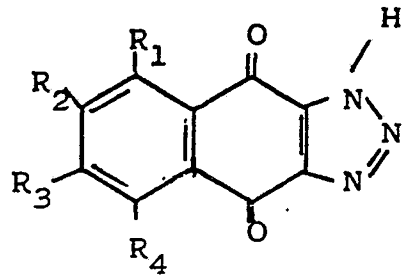

(I)

or a pharmaceutically acceptable salt thereof wherein $R_1$, $R_2$, $R_3$ and $R_4$ which may be the same or different represent hydrogen, halogen, nitro, lower alkyl or lower alkoxy, where lower means of up to six carbon atoms or any adjacent two of $R_1$ to $R_4$ taken together represent an alkylene group containing from 3 to 5 carbon atoms or a 1,4-buta-1,3-dienylene group, characterised by deprotecting a compound of formula (II):

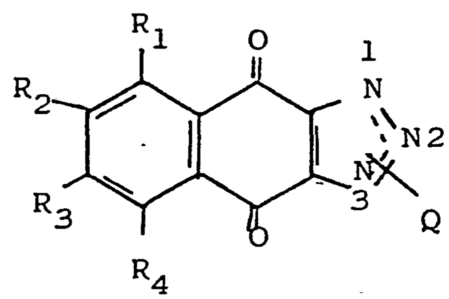

wherein Q is a N-protecting group at N-1 or N-3 and the other variables are as defined; and optionally thereafter salifying the compound of the formula (I) so formed.

2. A process according to claim 1, characterised in that Q is 4-methoxybenzyl.

3. A process according to claim 1 or 2, characterised in that Q is removed by acid catalysis.

4. A process according to claim 3, characterised in that acid used is trifluoroacetic acid.

5. A process according to any one of claims 1 to 4, characterised in that $R_1$ and $R_4$ are hydrogen and $R_2$ and $R_3$ are the same or different and are methyl, ethyl or n-propyl.

6. A process according to claim 5, characterised in that the compound of the formula (I) is 4,9-dihydro-6,7-dimethyl-4,9-dioxo-1H-naphtho[2,3-d]-v-triazole.

7. A process according to claim 5, characterised in that the process is for the preparation of the sodium salt of the compound of claim 6.

8. A compound of the formula (II):

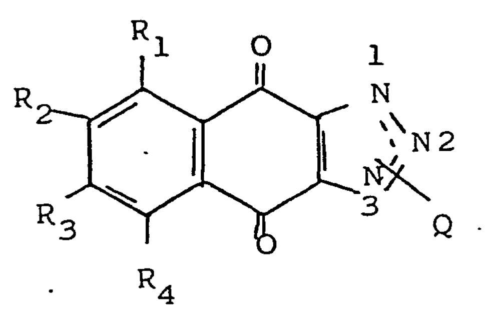

(II)

wherein Q is a N-protecting group at N-1 or N-3 and the other variables are as defined.

9. A compound according to claim 8, characterised in that Q is 4-methoxy,2,4-dimethoxybenzyl or 2,4,6-trimethoxybenzyl.